(19) **Europäisches Patentamt**

**European Patent Office**

**Office européen des brevets**

(11) **EP 1 449 540 A1**

(12) **EUROPEAN PATENT APPLICATION**
published in accordance with Art. 158(3) EPC

(43) Date of publication:
**25.08.2004 Bulletin 2004/35**

(51) Int Cl.⁷: **A61K 45/00**, A61K 47/48,
A61K 47/32, A61K 47/34,
A61K 47/38, A61K 35/74,
A61P 43/00

(21) Application number: **02779959.2**

(22) Date of filing: **31.10.2002**

(86) International application number:
**PCT/JP2002/011335**

(87) International publication number:
**WO 2003/037375 (08.05.2003 Gazette 2003/19)**

(84) Designated Contracting States:
**AT BE BG CH CY CZ DE DK EE ES FI FR GB GR
IE IT LI LU MC NL PT SE SK TR**

(30) Priority: **02.11.2001 JP 2001338016**

(71) Applicant: **SEKISUI CHEMICAL CO., LTD.
Osaka-shi, Osaka 530-8565 (JP)**

(72) Inventors:
• **SHINMURA, Kazuo,
SEKISUI CHEMICAL CO., LTD.
Mishima-gun, Osaka 618-8589 (JP)**

• **ABE, Yoshiko, SEKISUI CHEMICAL CO., LTD.
Mishima-gun, Osaka 618-8589 (JP)**
• **KURIYAMA, Kiyoshi,
SEKISUI CHEMICAL CO., LTD.
Mishima-gun, Osaka 618-8589 (JP)**

(74) Representative: **Merkle, Gebhard et al
TER MEER STEINMEISTER & PARTNER GbR,
Patentanwälte,
Mauerkircherstrasse 45
81679 München (DE)**

(54) **CYTOKINE-INDUCING MATERIAL AND CYTOKINE-INDUCING INSTRUMENT**

(57)    A cytokine-inducing material and a cytokine-inducing instrument to be used in cytokine-induction therapy and the like. More specifically speaking, a cytokine-inducing material characterized by containing a water-insoluble induction potentiator comprising a cytokine-inducer such as BCG, apolymer, etc.; and a cytokine-inducing instrument having this inducing material packed in a container. By bringing the inducing material into contact with, for example, blood or blood components, cytokine can be induced more effectively than conventional cases.

FIG. 3

**Description**

**TECHNICAL FIELD**

The present invention relates to cytokine-inducing material and instrument for use in the cytokine-inducing therapy or the like, more particularly to cytokine-inducing material and instrument which enable effective induction of cytokines.

**BACKGROUND ART**

Cytokine is a general term for a diversity of factors of cell signal transductions. Examples of cytokines include interferon-α, interferon-β, interferon-γ (IFN-γ), interleukin 1, interleukin 18, tumor necrosis factor-α (Tumor Necrosis Factor-α, TNF-α), tumor necrosis factor-β (Tumor Necrosis Factor-β), transforming growth factor-α (Transforming Growth Factor-α), transforming growth factor-β (Transforming Growth Factor-ß, TGF-ß) and various cell growth factors (Special 1995 number of Clinical Immunity Vol. 27, "All about cytokines", Kagaku Hyoron-sha, Clinical Immunity Vol. 36, 39 - 44, 2001).

Cytokine is known to exhibit various activities in vivo and be involved in various diseases. A cytokine- inducing therapy has been conventionally conducted which causes such activities of cytokine in vivo to thereby treat for diseases. In the cytokine-inducing therapy, a cytokine inducer is dosed to a patient to cause induction of cytokine in vivo. Various materials are known to serve as material inducing cytokine for use in such a cytokine-inducing therapy. Examples of known materials inducing cytokine include microorganism-derived materials such as OK-432, Bacillus Callete Guerin (BCG), Bestatin, Maruyama vaccine and romurtide, and basidiomycetes- derived materials such as Krestin, lentinan and sizofiran.

For example, OK-432, BCG and the like are known to induce cytokines, such as interleukin 1 and interferon-γ, from blood (Gifu University Medical Report 43: 166 - 177, 1995, Molecular Medicine Vol.36, extra edition, 220 - 229, 1999).

Although possible to induce cytokines in vivo, the above-described cytokine-inducing therapy is hard to induce cytokines in a sufficient amount and is accordingly difficult to provide their strong efficacies, which has been a problem. A dosage of a cytokine inducer may be increased to effectively induce cytokines. However, the increased dosage heightens side effects to result in the failure to achieve effective therapy.

Japanese Patent Laying-Open No. Sho 60-120821 describes a leukocyte stimulator for treatment of malignant tumor, which comprises a stimulating agent covalently bonded to an insoluble carrier. Also, Japanese Patent Laying-Open No. Sho 61-277628 describes a leukocyte stimulator for treatment of cancer, which comprises interleukin 1, OK-432, recombinant interleukin 2 or interferon-γ covalently bonded to an insoluble carrier. These leukocyte stimulators induce tumor cytotoxic cells. These prior art references provide no description as to cytokine induction.

**DISCLOSURE OF THE INVENTION**

In view of the current state of the above-described prior art, it is an object of the present invention to provide novel cytokine-inducing material and cytokine-inducing instrument which enable establishment of a more effective cytokine-inducing therapy relative to conventional cytokine-inducing therapies.

The cytokine-inducing material of the present invention contains a cytokine-inducing agent and a water-insoluble induction enhancer.

The inventors of this application have completed the present invention as a consequence of their finding that a cytokine-inducing material, either containing a cytokine-inducing agent and a water-insoluble induction enhancer or comprising an insoluble induction enhancer incorporating a cytokine-inducing agent fixed thereto, has the capability to induce a markedly large amount of cytokines.

The cytokine-inducing instrument of the present invention has a container, and a cytokine-inducing material constituted according to the present invention and accommodated in the container.

The present invention is below described in detail.

Although not limiting, the induction enhancer in the present invention comprises a water-insoluble material, either metallic, organic or inorganic. The induction enhancer preferably comprises a water-insoluble organic material, more preferably a water-insoluble polymeric material.

Examples of metallic induction enhancer include metals such as gold, gold alloys, silver, silver alloys, titanium, titanium alloys and stainless steel.

Examples of inorganic induction enhancers include active carbon, glass, glass derivatives, silica-based compositions, alumina and hydroxyapatite.

Examples of organic or polymeric induction enhancers include celluloses, agaroses, dextrans, polystyrenes, acrylic esters, polyethylene terephthalates, nylons, polyvinyl alcohols, polysulfons, polyamides, polyacrylonitriles, polyethylenes, polyurethanes, polypropylenes and polyesters.

Examples of polystyrenes include divinyl benzene-styrene copolymers. Examples of acrylic esters include polymethyl methacrylate and polyhydroxyethyl methacrylate. Particularly preferred are polymericmaterials such as based on polystyrenes, acrylic esters, nylons, polyesters, celluloses and polyvinyl alcohols.

The induction enhancer is nonpolar and may be hydrophobic. In this case, a polystyrene-based polymeric material can be used for the induction enhancer. Such an induction enhancer can be hydrophilicized at its surface as by surface modification or surface coating.

The shape or form of the induction enhancer is not particularly specified. The induction enhancer may have a generally-known form, such as a fiber, non-woven, sponge, particulate, film or hollow fiber.

The induction enhancer, if particulate, preferably has a size between 50 μm and 2 mm and, if fibrous, preferably has a diameter of up to 10 μm, more preferably up to 5 μm. If a fibrous form is selected, the induction enhancer may preferably comprise a non-woven fabric. In such a case, it is particularly preferred that a constituent fiber has a diameter of up to 3 μm.

It is particularly preferred that the induction enhancer comprises a leukocyte adsorbent material. Examples of useful leukocyte adsorbent materials include polymeric materials such as based on polystyrenes, acrylic esters, polyesters, nylons, polyvinyl alcohols and cellulose based materials, e.g., cellulose acetate; and glass-based materials.

Also preferable for use as the induction enhancer are materials having a surface roughness imparted thereto. Such materials preferably have a surface irregularity, as specified by a centerline average roughness Ra value within the range of 0.2 μm - 10 μm and a mean spacing Sm value of unevenness within the range of 5 μm - 200 μm.

The Ra value refers to a centerline average roughness according to JIS B 0601-1982. The mean spacing Sm value of unevenness is defined as follows.

Mean Spacing Sm Value of Unevenness

The latest JIS provides a standard as to an information of surface roughness in the height direction but provides no standard as to an information of surface roughness in the planar direction. However, the unevenness in the present invention can also be specified by the spacings of irregularity in the planar direction, as will become apparent from the below-given Examples. Accordingly, the present invention utilizes the mean spacing Sm value of unevenness in specifying the degree of irregularity in the planar direction.

The mean spacing Sm value of unevenness is given as follows.

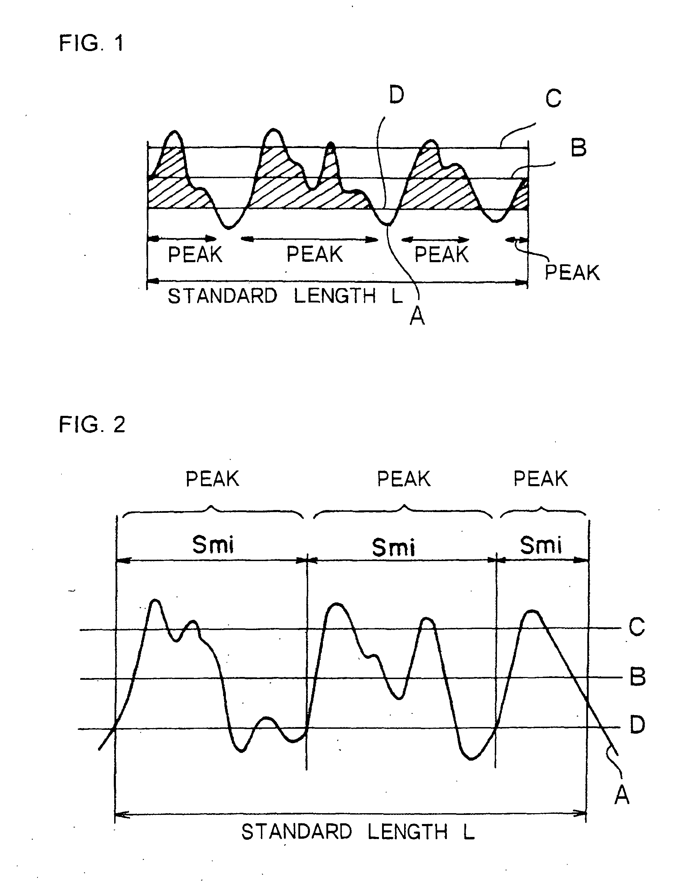

First, an upper count level C and a lower count level D are drawn to locate above and below a center line B of a roughness curve A shown in Figure 1 at a determined interval. When one or more intersections of the upper count level C and the roughness curve A exist between adjacent two points at which the lower count level D crosses the roughness curve A, "peak" is defined as a unit peak.

As shown in Figure 2, a centerline length of each peak which exists in a standard length L is given by $Sm_i$. Then, a mean spacing Sm value of unevenness is given by the following equation (1).

$$Sm = \frac{1}{n} \sum_{i=1}^{n} Sm\, i$$

(n: number of peaks) $\cdots$ (1)

That is, the mean spacing Sm value of unevenness indicates a mean value of spacing of peaks present in the standard length L. As such, the conditions of irregularity along the planar direction can be defined by the mean spacing Sm value of unevenness.

The surface roughness may be attributed to the porosity of the induction enhancer. Porous polymeric materials such as based on polystyrenes, acrylic esters, polyesters, nylons, celluloses and polyvinyl alcohols are suitable for use as the induction enhancer.

Alternatively, the surface roughness may be attributed to the fibrous form of the material used. Fibrous or non-woven materials are suitable for use as the induction enhancer. Polymeric materials in the fibrous or non-woven form are particularly preferred. Examples include fibrous and non-woven polymeric materials such as composed of polystyrenes, acrylic esters, polyesters, nylons, celluloses and polyvinyl alcohols.

As stated above, induction of cytokines are remarkably enhanced by imparting a proper surface roughness, at least 0.2 μm in terms of an Ra value, to the induction enhancer. The size of a leukocyte is 10 μm - 20 μm. When these facts are taken into consideration, it is preferred that the induction enhancer has an Ra value of 0.2 μm - 10 μm. Because the specified Ra value is much smaller than the size of a leukocyte, it does not seem that the cytokine induction enhancing effect of such surface roughness simply results from the increase of a contact area.

Examples of cytokine-inducing agents include bacteria and their components such as BCG, BCG-CWS, PPD (Purified protein derivatives Tuberculin), Nocardia-CWS, OK-432 and Muramyldipeptide; polysaccharides such as PSK (Klestin), lentinan and sizofiran; polymers such as poly I:C and poly A: U; and chemical substances such as Levamisole, DNCB, Azimexon, Tilorone and Bestatin. Besides the above physiologically active substances, a variety of substances can also be used as the cytokine-inducing agent, including bacteria, components of bacteria, peptides, nucleic acids, proteins, sugars and lipids.

Among the above-listed substances, bacteria and substances derived from such bacteria are preferred for use as the cytokine-inducing agent.

Acid-fast bacteria and substances derived from acid-fast bacteria are also preferred for use as the cytokine-inducing agents. Among them, tubercule bacilli and substances derived from tubercule bacilli are particularly preferred for use as the cytokine-inducing agent. BCG, an attenuated strain of mycobacterium bovis, and substances derived from BCG are also particularly preferred.

Also, hemolytic streptococci and substances derived from hemolytic streptococci are preferred for use as the cytokine-inducing agent.

Also, actinomycetes and substances derived from actinomycetes are preferred for use as the cytokine-inducing agent.

Some of the above cytokine-inducing agents, if alone, may be difficult to fully exhibit cytokine-inducing capabilities. However, they can exhibit their cytokine-inducing activities, when used in combination with the insoluble induction enhancer. This accordingly permits the use of various substances, other than the conventionally-used cytokine-inducing substances, as the cytokine-inducing agent in the present invention.

Various techniques generally known in the art, such as physical adsorption, covalent bonding and ionic bonding, can be utilized to fix the cytokine-inducing agent onto a surface of the induction enhancer. In the case of covalent bonding or the like, a spacer having an optional length may be introduced at a location where the cytokine-inducing agent is to be bonded to the induction enhancer, if necessary.

The cytokine-inducing agent, if comprised as of bacteria, may be optionally subjected to various pretreatments such as wash, fractionating and grinding operations of bacteria, before it is fixed. The cytokine-inducing agent, if comprised as of viable bacteria, can be subjected to various methods, e. g. , heat, chemical, radiation and gas sterilization treatments, either before or during or after it is fixed, to kill those bacteria. The heat treatment can be illustrated by an autoclaving treatment. Examples of chemical treatments include glutaric aldehyde, formalin and ethanol treatments. The radiation and gas sterilization treatments can be illustrated by a γ-ray treatment and an ethylene oxide gas treatment, respectively.

The cytokine-inducing agent comprised of a microorganism such as BCG can be fixed to the induction enhancer by the bonding of amino acid or saccharic substances, which are contained in outer surface walls of bacteria, to a functional group in the induction enhancer such as a carboxylic, amino and/or epoxy group. During this operation, a spacer may be introduced having various chain lengths and structures, when necessary.

When an outer layer of a microorganism such as BCG is covered as with a lipid, this lipid may be optionally removed by wash, before the fixation is carried out.

The preferred fixation technique is a physical adsorption technique. The cytokine-inducing agent can be fixed onto the induction enhancer by the physical adsorption technique. Particularly when the induction enhancer has a hydrophobic surface, physical adsorption can be utilized effectively to fix BCG or the like cytokine-inducing agent onto such an induction enhancer. The cytokine-inducing agent, if comprised of a microorganismor its components, may carry a charge at its surface. In this case, such a cytokine-inducing agent can be fixed to the induction enhancer having an oppositely charged surface by physical adsorption.

The usage of the induction enhancer is not particularly specified. When the induction enhancer is used in a particulate form, a bulk volume of the induction enhancer is generally in the approximate range of 0.02% - 80%, preferably 0.1 % - 50 %, relative to a blood volume.

The amount of the cytokine-inducing agent used is not particularly specified. For example, the cytokine-inducing agent, if comprising BCG, is preferably added to blood in the concentration of 0.001 mg - 10 mg/mL, on a dry weight basis, and, if comprising OK-432, is preferably added to blood in the concentration of 0.0001 KE - 10 KE/mL.

When in use of the cytokine-inducing instrument in accordance with the present invention, the cytokine-inducing material, which contains the'induction enhancer and cytokine-inducing agent, is allowed to contact with blood, a blood component or the like whereby cytokines are induced effectively in the blood, blood component or the like. In this case, a contact temperature is preferably maintained within the range of 15 - 42°C. This enables more effective induction of cytokines.

The induction enhancer and cytokine-inducing agent may be mixed together before they are allowed to contact with blood. Alternatively, they may be separately allowed to contact with the blood, blood component or the like.

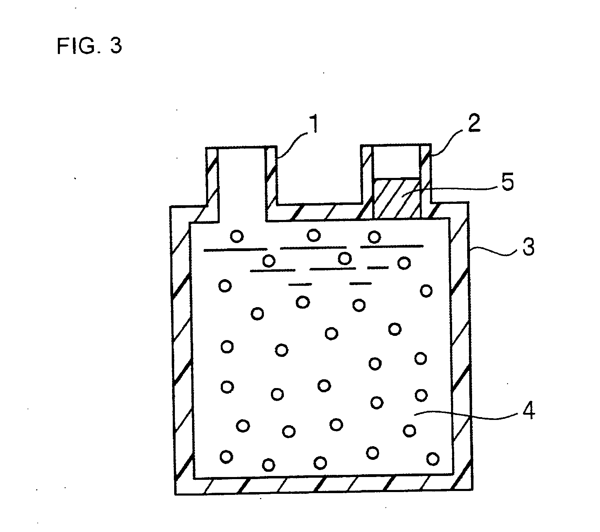

In the present invention, a construction of the container used to accommodate the cytokine-inducing material is not particularly specified. As schematically shown in Figure 3, the container 3 preferably has an inlet 1 for introducing blood or the like and an outlet 2 from which blood 4 or the like, subsequent to contact with the cytokine-inducing material, is guided to an outside of the container.

It is particularly preferred that the container used to accommodate the cytokine-introducing material is constructed in the form of a column, a blood bag or the like.

More preferably, the cytokine-inducing instrument has a mechanism for preventing outflow of cytokine-inducing material, when blood is allowed to contact with the cytokine-inducing material and then guided to an outside of the container.

As schematically shown in Figure 3, the mechanism 5 for preventing outflow of cytokine-inducing material may be fixedly mounted within the container so as to prevent outflow of the cytokine-inducing material. Themechanism may beprovided with a separation membrane or filter. Also, the cytokine-inducing material may be separated from blood by centrifuging.

If a therapeutic need arises, a plasma or serum component may be separated from blood after it is contacted with the cytokine-inducing material.

Specifically, the cytokine-inducing material containing the induction enhancer in a particulate, fibrous or nonwoven form or the like is packed blood bag having an inlet and an outlet. Blood, a blood component or the like is passed through the inlet into the bag. If necessary, the blood, blood component or the like after induction of cytokines may be removed through the outlet for use. The blood bag preferably has a volume of 50 mL - 1, 000 mL, particularly preferably 100 mL - 400 mL. Such a blood bag preferably constitutes the cytokine-inducing instrument by accommodating a particulate, fibrous or nonwoven cytokine-inducing material.

Examples of particularly important cytokines include interferon- $\gamma$ (IFN-$\gamma$), interleukin 2 (IL-2), interleukin 10 (IL-10) , interleukin 12 (IL-12), tumor necrosis factor-$\alpha$ (Tumor Necrosis Factor-$\alpha$, TNF-$\alpha$) and transforming growth factor-$\beta$ (TGF-$\beta$). For example, IFN-$\gamma$ is a cytokine which plays a very important role in immune diseases such as rheumatism, inflammatory diseases, allergic diseases, cancers and other various diseases and can be expected as having a therapeutic effect on these diseases.

The above-described cytokine-inducing material and cytokine-inducing instrument can induce cytokines not only from blood, a blood component or the like but also from various cytokine-producing cells, examples of which include cells collected from tissues such as of bone marrow-derived cells, epidermal cells, fibroblasts, hepatocytes, osteoblasts, blood stem cells, embryonic stem cells, cultured cells and established cells.

**BRIEF DESCRIPTION OF THE DRAWINGS**

Figure 1 is a schematic view which explains surface roughness and "peaks" of irregularities;
Figure 2 is a schematic view which explains a mean spacing Sm value of irregularities for surface roughness; and
Figure 3 is a schematic sectional view which shows an embodiment of a cytokine-inducing instrument in accordance with the present invention.

**BEST MODE FOR CARRYING OUT THE INVENTION**

The following examples illustrate the present invention more specifically but are not intended to be limiting thereof.

In the following examples, IFN-$\gamma$, TNF-$\alpha$, IL-2, IL-10 and IL-12 in human plasma and rat plasma were quantified by using an R&D Systems ELISA kit, an Endogen ELISA kit and a Genzyme Techne ELISA kit. TGF-$\beta$ in human plasma was quantitatively determined by using a Promega ELISA kit.

(EXAMPLE 1)

An induction enhancer -1 (synthetic aromatic adsorbent, product of Mitsubishi Chemical Corp. , product name : DIAION HP-50). was washed via decantation with purified water (product of Otsuka Pharmaceutical Co., Ltd.) and then with methanol (product of Wako Pure Chemicals Industries, Ltd., for HPLC use). The induction enhancer-1 was thereafter washed via decantation with physiological saline for injection (product of Otsuka Pharmaceutical Co., Ltd.). The induction enhancer-1 in the particle bulk volume of 50 $\mu$L was then packed in a sterilized tube (product of DIATRON Corp., Eppendorf tube for 1.5 ml use).

Blood was collected from a healthy human to prepare venous blood containing 15 IU/ml of heparin. BCG (product of Japan BCG Manufacturing Co., Ltd.) was added in the concentration of 1 mg/mL of blood. Here, BCG was prepared with physiological saline. A ratio by volume of the physiological saline to blood was brought to 1.25 %.

About 1.45 mL of the BCG-containing blood was introduced into the tube packed with the induction enhancer-1.

The tube was tumbled to stir the blood and then attached to a rotary mixer (product of Taitech Corp.) which was subsequently rotated at 6 rpm in a constant temperature vessel to effect incubation at 37°C for 24 hours. After incubation, the blood was centrifuged at 3, 500 rpm (product of Tomy Seiko Co., Ltd., micro high-speed centrifuge MRX-150) at 4 °C for 15 minutes. Blood plasmas were then collected from the blood for cryopreservation at -20 °C. The preserved plasmas were then melted and the amount of IFN-γ induced in the plasmas was determined using a Human IFN-γ ELISA kit (product of R&D Systems or ENDOGEN). The results are shown in the following Table 1.

(COMPARATIVE EXAMPLE 1)

The induction enhancer-1 was excluded and the BCG-incorporated blood was used in the amount of 1.5 mL. Otherwise, the procedure of Example 1 was followed to collect the plasmas and determine the amount of IFN-γ induced in the plasmas. The results are shown in the following Table 1.

(COMPARATIVE EXAMPLE 2)

The procedure of Example 1 was followed, except that BCG was not added to blood, to determine the amount of IFN-γ induced. The results are shown in the following Table 1.

Table 1

| | Induction Enhancer 1 Bulk Volume (μL) | Concentration of BCG (mg/mL) | Amount of IFN-γ Induced (pg/mL) |
|---|---|---|---|
| Ex.1 | 50 | 1 | 162 |
| Comp.Ex.1 | - | 1 | 38 |
| Comp.Ex.2 | 50 | - | <10 |

(EXAMPLE 2)

The duration of incubation at 37 °C was altered from 24 hours to 4 hours. Otherwise, the procedure of Example 1 was followed to obtain cryopreserved plasmas. The cryopreserved plasmas were then melted and the amount of TNF-α induced in the plasmas was determined using a Human TNF-α ELISA kit (product of R&D System). The results are shown in the following Table 2.

(COMPARATIVE EXAMPLE 3)

The procedure of Example 2 was followed, except that the induction enhancer-1 was excluded and the BCG-incorporated blood was used in the amount of 1.5 mL, to determine the amount of TNF-α induced. The results are shown in the following Table 2.

(COMPARATIVE EXAMPLE 4)

The procedure of Example 2 was followed, except that BCG was not added to blood, to determine the amount of TNF-α induced. The results are shown in the following Table 2.

Table 2

| | Induction Enhancer 1 Bulk Volume (μL) | Concentration of BCG (mg/mL) | Amount of TNF-α Induced (ng/mL) |
|---|---|---|---|
| Ex.2 | 50 | 1 | 4.7 |
| Comp.Ex.3 | - | 1 | 1.1 |
| Comp.Ex.4 | 50 | - | <0.8 |

(EXAMPLE 3)

The bulk volume of the induction enhancer-1 was altered from 50 µL to 5 µL and the blood was added in the amount of 1.495 mL. Otherwise, the procedure of Example 1 was followed to determine the amount of IFN-γ induced in the plasmas. The results are shown in the following Table 3.

(EXAMPLE 4)

The bulk volume of the induction enhancer-1 was altered from 50 µL to 10 µL and the blood was added in the amount of 1.49 mL. Otherwise, the procedure of Example 1 was followed to determine the amount of IFN-γ induced in the plasmas. The results are shown in the following Table 3.

(EXAMPLE 5)

The bulk volume of the induction enhancer-1 was altered from 50 µL to 20 µL and the blood was added in the amount of 1.48 mL. Otherwise, the procedure of Example 1 was followed to determine the amount of IFN-γ induced in the plasmas. The results are shown in the following Table 3.

(EXAMPLE 6)

The procedure of Example 1 was followed to determine the amount of IFN-γ induced in the plasmas. The results are shown in the following Table 3.

(EXAMPLE 7)

The bulk volume of the induction enhancer-1 was altered from 50 µL to 200 µL and the blood was added in the amount of 1.3 mL. Otherwise, the procedure of Example 1 was followed to determine the amount of IFN-γ induced in the plasmas. The results are shown in the following Table 3.

(COMPARATIVE EXAMPLE 5)

The induction enhancer-1 was excluded and the BCG-incorporated blood was used in the amount of 1.5 mL. Otherwise, the procedure of Example 3 was followed to determine the amount of IFN-γ induced. The results are shown in the following Table 3.

(COMPARATIVE EXAMPLE 6)

The procedure of Example 3 was followed, except that BCG was not added to blood, to determine the amount of IFN-γ induced. The results are shown in the following Table 3.

(COMPARATIVE EXAMPLE 7)

The procedure of Example 4 was followed, except that BCG was not added to blood, to determine the amount of IFN-γ induced. The results are shown in the following Table 3.

(COMPARATIVE EXAMPLE 8)

The procedure of Example 5 was followed, except that BCG was not added to blood, to determine the amount of IFN-γ induced. The results are shown in the following Table 3.

(COMPARATIVE EXAMPLE 9)

The procedure of Example 6 was followed, except that BCG was not added to blood, to determine the amount of IFN- y induced. The results are shown in the following Table 3.

(COMPARATIVE EXAMPLE 10)

The procedure of Example 7 was followed, except that BCG was not added to blood, to determine the amount of IFN- y induced. The results are shown in the following Table 3.

Table 3

| | Induction Enhancer 1 Bulk Volume (µL) | Concentration of BCG (mg/mL) | Amount of IFN-γ Induced (pg/mL) |
|---|---|---|---|
| Ex.3 | 5 | 1 | 115 |
| Ex.4 | 10 | 1 | 152 |
| Ex.5 | 20 | 1 | 297 |
| Ex.6 | 50 | 1 | 573 |
| Ex.7 | 200 | 1 | 453 |
| Comp.Ex.5 | - | 1 | 104 |
| Comp.Ex.6 | 5 | - | <10 |
| Comp.Ex.7 | 10 | - | <10 |
| Comp.Ex.8 | 20 | - | <10 |
| Comp.Ex.9 | 50 | - | <10 |
| Comp.Ex.10 | 200 | - | <10 |

(EXAMPLE 8)

The procedure of Example 1 was followed, except that the bulk volume of the induction enhancer-1 was altered to 20 µL, to prepare the tube packed with the induction enhancer-1.

Picibanil (product of Chugai Pharmaceutical Co., Ltd., OK-432), in place of BCG, was added to each blood sample in a concentration of 0.1 KE/mL. This OK-432 was prepared using an RPMI medium. The ratio of the RPMI medium to blood was 20 %. The OK-432 incorporated blood was poured into the tube packed with a 20 µL bulk volume of the induction enhancer-1 to a tube scale of 1.5 mL. That is, the blood was added in the amount of about 1.48 mL.

Then, the amount of IFN-γ induced was determined in the same manner as in Example 1. The results are shown in the following Table 4.

(EXAMPLE 9)

The bulk volume of the induction enhancer-1 was altered to 50 µL and the OK-432 incorporated blood was used in the amount of 1.45 mL. Otherwise, the procedure of Example 8 was followed to determine the amount of IFN-γ induced. The results are shown in the following Table 4.

(EXAMPLE 10)

The bulk volume of the induction enhancer-1 was altered to 100 µL and the OK-432 incorporated blood was used in the amount of 1.40 mL. Otherwise, the procedure of Example 8 was followed to determine the amount of IFN-γ induced. The results are shown in the following Table 4.

(COMPARATIVE EXAMPLE 11)

The induction enhancer-1 was excluded and the blood incorporated OK-432 was used in the amount of 1. 5mL. Otherwise, the procedure of Example 8 was followed to determine the amount of IFN-γ induced. The results are shown in the following Table 4.

(COMPARATIVE EXAMPLE 12)

The procedure of Example 8 was followed, except that Picibanil (OK-432) was not added, to determine the amount of IFN-γ induced. The results are shown in the following Table 4.

(COMPARATIVE EXAMPLE 13)

The procedure of Example 9 was followed, except that Picibanil (OK-432) was not added, to determine the amount of IFN-γ induced. The results are shown in the following Table 4.

(COMPARATIVE EXAMPLE 14)

The procedure of Example 10 was followed, except that Picibanil (OK-432) was not added, to determine the amount of IFN-γ induced. The results are shown in the following Table 4.

Table 4

|  | Induction Enhancer 1 Bulk Volume (μL) | Concentration of OK-432 (KE/mL) | Amount of IFN-γ Induced (pg/mL) |
|---|---|---|---|
| Ex.8 | 20 | 0.1 | 360 |
| Ex.9 | 50 | 0.1 | 367 |
| Ex.10 | 100 | 0.1 | 323 |
| Comp.Ex.11 | - | 0.1 | 114 |
| Comp.Ex.12 | 20 | - | <10 |
| Comp.Ex.13 | 50 | - | <10 |
| Comp. Ex.14 | 100 | - | <10 |

(EXAMPLE 11)

An induction enhancer-2 (synthetic acrylic ester resin, product of Organo Corp., product number: AMBERLITE XAD-7), in place of the induction enhancer-1, was used. Otherwise, the procedure of Example 1 was followed to determine the amount of IFN-γ induced in the plasmas. The results are shown in the following Table 5.

(EXAMPLE 12)

The bulk volume of the induction enhancer-2 was altered from 50 μL to 200 μL and the BCG-incorporated blood was used in the amount of 1.3 mL. Otherwise, the procedure of Example 11 was followed to determine the amount of IFN-γ induced. The results are shown in the following Table 5.

(COMPARATIVE EXAMPLE 15)

The induction enhancer-2 was excluded and the BCG-incorporated blood was used in the amount of 1.5 mL. Otherwise, the procedure of Example 11 was followed to determine the amount of IFN-γ induced. The results are shown in the following Table 5.

(COMPARATIVE EXAMPLE 16)

The procedure of Example 11 was followed, except that BCG was not added to blood, to determine the amount of IFN-γ induced. The results are shown in the following Table 5.

(COMPARATIVE EXAMPLE 17)

The procedure of Example 12 was followed, except that BCG was not added to blood, to determine the amount of I FN- y induced. The results are shown in the following Table 5.

Table 5

|  | Induction Enhancer 2 Bulk Volume (μL) | Concentration of BCG (mg/mL) | Amount of IFN-γ Induced (pg/mL) |
|---|---|---|---|
| Ex.11 | 50 | 1 | 299 |

Table 5   (continued)

|  | Induction Enhancer 2 Bulk Volume (μL) | Concentration of BCG (mg/mL) | Amount of IFN-γ Induced (pg/mL) |
|---|---|---|---|
| Ex.12 | 200 | 1 | 218 |
| Comp.Ex.15 | - | 1 | 104 |
| Comp.Ex.16 | 50 | - | <12 |
| Comp. Ex.17 | 200 | - | <12 |

The following Examples and Comparative Examples illustrate induction of cytokines in rat blood.

(EXAMPLE 13)

The procedure of Example 1 was followed to obtain the sterilized tube packed with a 50 μL bulk volume of the induction enhancer-1.

Venous blood containing 15 IU/ml of heparin was collected from a Wister rat (7 weeks old, male, purchased from Japan SLC, Inc.). Picibanil (product of Chugai Pharmaceutical Co., Ltd., OK-432) was added to the blood in the concentration of 0.1 KE/mL. This OK-432 was prepared using an RPMI medium. The ratio of the RPMI medium to blood was 20 %. The OK-432 incorporated blood was added to the tube, packed with the induction enhancer-1, to a tube scale of 1.5 mL.

Subsequently, the procedure of Example 1 was followed to collect blood plasmas and determine the amount of IFN- y induced therein by using a Rat IFN-γ quantification kit (product of Genzyme Techne). The results are shown in the following Table 6.

(EXAMPLE 14)

The bulk volume of the induction enhancer-1 was altered to 500 μL and the OK-432 incorporated blood was used in the amount of 1.0 mL. Otherwise, the procedure of Example 13 was followed to determine the amount of IFN-γ induced. The results are shown in the following Table 6.

(COMPARATIVE EXAMPLE 18)

The induction enhancer-1 was excluded and the OK-432 incorporated blood was used in the amount of 1.5 mL. Otherwise, the procedure of Example 13 was followed to determine the amount of IFN-y induced. The results are shown in the following Table 6.

(COMPARATIVE EXAMPLE 19)

The procedure of Example 13 was followed, except that OK-432 was not added to the blood, to determine the amount of IFN-γ induced. The results are shown in the following Table 6.

(COMPARATIVE EXAMPLE 20)

The procedure of Example 14 was followed, except that OK-432 was not added to the blood, to determine the amount of IFN-γ induced. The results are shown in the following Table 6.

Table 6

|  | Induction Enhancer 1 Bulk Volume (μL) | Concentration of OK-432 (KE/mL) | Amount of IFN-γ Induced (pg/mL) |
|---|---|---|---|
| Ex.13 | 50 | 0.1 | 251 |
| Ex.14 | 500 | 0.1 | 435 |
| Comp.Ex.18 | - | 0.1 | 72 |
| Comp.Ex.19 | 50 | - | <40 |

Table 6 (continued)

| | Induction Enhancer 1 Bulk Volume (μL) | Concentration of OK-432 (KE/mL) | Amount of IFN-γ Induced (pg/mL) |
|---|---|---|---|
| Comp.Ex.20 | 500 | - | <40 |

(EXAMPLE 15)

The procedure of Example 1 was followed to obtain the sterilized tube packed with a 50 μL bulk volume of the induction enhancer-1.

Blood was collected from a Wister rat (7 weeks old, male, purchased from Japan SLC, Inc.) to obtain venous blood containing 15 IU/ml of heparin. BCG was added to this blood in the concentration of 1 mg/mL. This BCG was prepared using physiological saline. The ratio in volume of the physiological saline to the blood was 1.25 %. The BCG-incorporated blood was added to the tube, packed with the induction enhancer-1, to a tube scale of 1.45 mL.

Subsequently, the procedure of Example 1 was followed to collect blood plasmas and determine the amount of IFN- y induced therein by using a Rat IFN-γ quantification kit (product of Genzyme Techne). The results are shown in the following Table 7.

(EXAMPLE 16)

The bulk volume of the induction enhancer-1 was altered from 50 μL to 500 μL and the BCG-incorporated blood was used in the amount of 1.0 mL. Otherwise, the procedure of Example 15 was followed to determine the amount of IFN-γ induced. The results are shown in the following Table 7.

(COMPARATIVE EXAMPLE 21)

The induction enhancer-1 was excluded and the BCG-incorporated blood was used in the amount of 1.5 mL. Otherwise, the procedure of Example 15 was followed to determine the amount of IFN-γ induced. The results are shown in the following Table 7.

(COMPARATIVE EXAMPLE 22)

The procedure of Example 15 was followed, except that BCG was not added to the blood, to determine the amount of IFN-γ induced. The results are shown in the following Table 7.

(COMPARATIVE EXAMPLE 23)

The procedure of Example 16 was followed, except that BCG was not added to the blood, to determine the amount of IFN-γ induced. The results are shown in the following Table 7.

Table 7

| | Induction Enhancer 1 Bulk Volume (μL) | Concentration of BCG (mg/mL) | Amount of IFN-γ Induced (pg/mL) |
|---|---|---|---|
| Ex.15 | 50 | 1 | 132 |
| Ex.16 | 500 | 1 | 233 |
| Comp.Ex.21 | - | 1 | 68 |
| Comp.Ex.22 | 50 | - | <40 |
| Comp.Ex.23 | 500 | - | <40 |

(EXAMPLE 17)

The duration of incubation at 37 °C was altered from 24 hours to 4 hours. Otherwise, the procedure of Example 15 was followed to collect blood plasmas and determine the amount of TNF-α induced therein by using a Rat Tumor Necrosis Factor-α quantification kit (product of Genzyme Techne). The results are shown in the following Table 8.

(COMPARATIVE EXAMPLE 24)

The induction enhancer-1 was excluded and the BCG-incorporated blood was used in the amount of 1.5 mL. Otherwise, the procedure of Example 17 was followed to collect blood plasmas and determine the amount of TNF-$\alpha$ induced therein. The results are shown in the following Table 8.

(COMPARATIVE EXAMPLE 25)

The procedure of Example 17 was followed, except that BCG was not added to the blood, to determine the amount of TNF-$\alpha$ induced. The results are shown in the following Table 8.

Table 8

|  | Induction Enhancer 1 Bulk Volume ($\mu$L) | Concentration of BCG (mg/mL) | Amount of TNF-$\alpha$ Induced (ng/mL) |
|---|---|---|---|
| Ex.17 | 50 | 1 | 12.7 |
| Comp.Ex.24 | - | 1 | 2.1 |
| Comp.Ex.25 | 50 | - | < 1.2 |

(EXAMPLES 18 - 23)

Induction Enhancer-3: A CA film (product of Artplus Corp., cellulose acetate film, product name: ACETYFILM VR-R) was used. Aplasticizer was extracted from the film by soxhlet extraction for 24 hours in methyl alcohol. After removal, the film was air dried for 15 hours and further dried at 80°C for 5 hours. Thereafter, the CA film was polished, using a Struers (Denmark) automaticpolisher (product name: PLANOPOL-PEDEMAX) witha220-, 500-, 1200-, 2400- or 4000-mesh sandpaper secured thereto, to provide a polished CA film having surface roughness on its both surfaces. This film was washed with methyl alcohol and then subdivided to 2.5 mm $\times$ 2.5 mm pieces. These pieces, measuring a bulk volume of about 200 $\mu$L, were washed with physiological saline for injection and then packed in a sterilized tube for 1.5 mL use.

Blood was collected from a healthy human to prepare venous blood containing 15 IU/ml of heparin. BCG (product of Japan BCG Manufacturing Co., Ltd.) was added to the blood in the concentration of 1 mg/mL. About 1.3 mL of the resulting blood was added to the tube. Subsequently, the amount of IFN-$\gamma$ induced was determined in the same manner as in Example 1. The results are shown in the following Table 9.

Table 9

|  | Example | | | | | |
|---|---|---|---|---|---|---|
|  | 18 | 19 | 20 | 21 | 22 | 23 |
|  | Unpolished | Polished | | | | |
|  |  | 4000-Mesh | 2400-Mesh | 1200-Mesh | 500-Mesh | 220-Mesh |
| Ra Value ($\mu$m) | 0.05 | 0.05 | 0.06 | 0.58 | 1.28 | 2.37 |
| Sm Value ($\mu$m) | 250 | 125 | 31 | 30 | 44 | 57 |
| Concentration of BCG (mg/mL) | 1 | 1 | 1 | 1 | 1 | 1 |
| Concentration of IFN-$\gamma$ (pg/mL) | 38 | 72 | 79 | 172 | 193 | 189 |

(EXAMPLES 24 - 29)

Induction Enhancer-4 : A PET film (product of Unitika Ltd., polyethylene terephthalate film, product name: EMBLET S-75) was used. The film was at its surfaces washed with methyl alcohol. Thereafter, this PET filmwas polished, using a Struers (Denmark) automaticpolisher (product name: PLANOPOL-PEDEMAX) with a 220-, 500-, 1200-, 2400- or 4000-mesh sandpaper secured thereto, to provide a polished CA film having surface roughness on its both surfaces. This film was washed with methyl alcohol and then subdivided to 2.5 mm x 2.5 mm pieces. These pieces, measuring a bulk volume of about 200 µL, were washed with physiological saline for injection and then packed in a sterilized tube for 1.5 mL use. Blood was collected from a healthy human to obtain venous blood containing 15 IU/ml of heparin. BCG (product of Japan BCG Manufacturing Co., Ltd.) was added to the blood in the concentration of 1 mg/mL. About 1.3 mL of the resulting blood was added to the tube. The procedure of Example 1 was then followed to determine the amount of IFN-γ induced. The results are shown in the following Table 10.

Table 10

| | Example | | | | | |
|---|---|---|---|---|---|---|
| | 24 | 25 | 26 | 27 | 28 | 29 |
| | Unpolished | Polished | | | | |
| | | 4000-Mesh | 2400-Mesh | 1200-Mesh | 500-Mesh | 220-Mesh |
| Ra Value (µm) | 0.07 | 0.12 | 0.19 | 0.61 | 1.55 | 2.24 |
| Sm Value (µm) | 475 | 37 | 126 | 31 | 47 | 77 |
| Concentration of BCG (mg/mL) | 1 | 1 | 1 | 1 | 1 | 1 |
| Concentration of IFN-γ (pg/mL) | 36 | 71 | 73 | 141 | 158 | 169 |

(EXAMPLES 30 - 35)

Induction Enhancer-5: A polystyrene film (product of Mitsubishi-Monsanto Co., Ltd., product name: SANTOCLEAR) was used. The film was at its surfaces washed with purified water. Thereafter, the polystyrene film was polished, using a Struers (Denmark) automatic polisher (product name: PLANOPOL-PEDEMAX) with a 220-, 500-, 1200-, 2400- or 4000-mesh sandpaper secured thereto, to provide a polished polystyrene film having surface roughness on its both surfaces. This film was washed with purified water and then subdivided to 2.5 mm x 2.5 mm pieces. These pieces, measuring a bulk volume of about 200 µL, were washed with physiological saline for injection and then packed in a sterilized tube for 1.5 mL use. Blood was collected from a healthy human to obtain venous blood containing 15 IU/ml of heparin. BCG (product of Japan BCG Manufacturing Co., Ltd.) was added to the blood in the concentration of 1 mg/mL. About 1.3 mL of the resulting blood was added to the tube. The procedure of Example 1 was then followed to determine the amount of IFN-γ induced. The results are shown in the following Table 11.

Table 11

| | Example | | | | | |
|---|---|---|---|---|---|---|
| | 30 | 31 | 32 | 33 | 34 | 35 |
| | Unpolished | Polished | | | | |
| | | 4000-Mesh | 2400-Mesh | 1200-Mesh | 500-Mesh | 220-Mesh |
| Ra Value (µm) | 0.06 | 0.08 | 0.21 | 0.72 | 1.75 | 2.44 |
| Sm Value (µm) | 375 | 52 | 43 | 27 | 39 | 71 |
| Concentration of BCG (mg/mL) | 1 | 1 | 1 | 1 | 1 | 1 |
| Concentration of IFN-γ (pg/mL) | 48 | 92 | 94 | 239 | 269 | 246 |

(EXAMPLE 36)

Cellulose acetate pellets (product of Artplus Corp., containing 30% acetyl triethyl citrate as a plasticizer) were injection molded to prepare 2.5 mm diameter spherical beads. The plasticizer was extracted from 50 g of these beads by soxhlet extraction at 50 °C for 24 hours in 300 mL methanol. Subsequent to extraction of the plasticizer, those beads were transferred onto a stainless-steel batting, air dried for 15 hours and then further dried at 80°C for 5 hours.

200 mL of such beads and an equal volume of an abrasive WHITE ABRAX (WA) #34 (product of Japan Abrasive Company) were introduced into a pot mill (product of Toyo Engineering Corp., product name: 51-ceramic pot mill BP-5). Also, several balls (product of Toyo Engineering Corp., product name: BB-13) for a ceramic pot mill were introduced. Polishing was applied for 5 hours by a ball polishing machine (pot mill manufactured by Nitto Kagaku Co., Ltd., product name: AN-3S) . As a result, beads were obtained having an Ra value of 1.36 μm and an Sm value of 97.2 μm (induction enhancer-6).

The obtained beads were washed three times with methanol and then five times with physiological saline for injection. These beads, measuring a bulk volume of 400 μL, were packed in a sterilized tube for 1.5 mL use in the same manner as in Example 1. The procedure of Example 1 was then followed, except that the blood was added in the amount of 1.1 mL, to determine the amount of IFN-γ induced. The results are shown in the following Table 12.

(EXAMPLE 37)

The procedure of Example 36 was followed, except that polishing was not-applied, to prepare the induction enhancer-6 having an Ra value of 0.186 μm and an Sm value of 298.7 μm. This induction enhancer was allowed to contact with the blood in the same manner as in Example 36. The results are shown in the following Table 12.

(COMPARATIVE EXAMPLE 26)

The induction enhancer-6 was excluded and the BCG-incorporated blood was used in the amount of 1.5 mL. Otherwise, the procedure of Example 36 was followed to determine the amount of IFN-γ induced. The results are shown in the following Table 12.

Table 12

|  | Centerline Average Roughness Ra (μm) | Mean Spacing of Unevenness Sm (μm) | Concentra-t ion of BCG (mg/mL) | Amount of IFN-γ Induced (pg/mL) |
|---|---|---|---|---|
| Ex.36 | 1.36 | 97.2 | 1 | 198 |
| Ex.37 | 0.19 | 298.7 | 1 | 101 |
| Comp.Ex.26 | - | - | 1 | 38 |

(EXAMPLES 38 - 42)

The procedure of Example 1 was followed, using the induction enhancer-1 (synthetic aromatic adsorbent, product of Mitsubishi Chemical Corp., product name: DIAION HP-50), the induction enhancer-2 (synthetic acrylic ester resin, product of Organo Corp., product number: AMBERLITE XAD-7), an induction enhancer-7 (synthetic aromatic resin, product of Organo Corp., product number: AMBERLITE XAD-2), an induction enhancer-8 (synthetic aromatic resin, product of Organo Corp., product number: AMBERLITE XAD-4) or an induction enhancer-9 (synthetic aromatic resin, product of Organo Corp., product number: AMBERLITE XAD-16HP). The results are shown in the following Table 13.

(COMPARATIVE EXAMPLE 27)

The induction enhancer was excluded and the BCG-incorporated blood was used in the amount of 1.5 mL. Otherwise, the procedure of Example 38 was followed. The results are shown in the following Table 13.

Table 13

|  | Induction Enhancer Bulk Volume (50 μL) | Material Type | Concentration of BCG (mg/mL) | Amount of IFN-γ Induced (pg/mL) |
|---|---|---|---|---|
| Ex.38 | Induction Enhancer 1 | HP50 | 1 | 299 |
| Ex.39 | Induction Enhancer 2 | XAD7 | 1 | 136 |
| Ex.40 | Induction Enhancer 7 | XAD2 | 1 | 283 |
| Ex.41 | Induction Enhancer 8 | XAD4 | 1 | 315 |
| Ex.42 | Induction Enhancer 9 | XAD16HP | 1 | 307 |
| Comp.Ex.27 | Absent | - | 1 | 61 |

(EXAMPLE 43)

BCG was suspended in physiological saline, diluted with a phosphate buffer and centrifuged at 4, 000 rpm for 15 minutes. After removal of a supernatant liquid, the resultant was again suspended in a phosphate buffer and centrifuged at 4,000 rpm (product of Tomy Seiko Co., Ltd., micro high-speed centrifuge MRX-150) for 15 minutes. This operation was repeated three times. The resultant was suspended in a phosphate buffer containing 50 % ethanol and then centrifuged at 4,000 rpm for 15 minutes. Subsequently, the resultant was suspended in ethanol and centrifuged at 4,000 rpm for 15 minutes. This operation was repeated twice. Wash with a phosphate buffer was again carried out three times. Such treated BCG in the amount of 2 mg was covalently bonded to the carboxyl-introduced induction enhancer-1 (bulk volume of 1 mL) by a carbodiimide process. The remaining reactive groups were reacted with ethanolamine. The resultant was washed with a phosphate buffer and then suspended in a phosphate buffer. The thus-obtained induction enhancer, measuring 100 μL, was packed in a sterilized tube.

BCG was not added in its individual form. The blood was added in the amount of 1.4 mL. Otherwise, the procedure of Example 1 was followed to determine the amount of IFN-γ induced. The results are shown in Table 14.

(COMPARATIVE EXAMPLE 28)

The procedure of Example 43 was followed, except that the treated BCG was not covalently bonded, to determine the amount of IFN-γ induced. The results are shown in Table 14.

(COMPARATIVE EXAMPLE 29)

The induction enhancer was not added and the BCG-incorporated (1 mg/mL) blood was used in the amount of 1.5 mL. Otherwise, the procedure of Example 43 was followed to determine the amount of IFN-γ induced. The results are shown in Table 14.

Table 14

|  | Induction Enhancer 1 Bulk Volume (100 μL) | Concentration of BCG (mg/mL) | Amount of IFN-γ Induced (pg/mL) |
|---|---|---|---|
| Ex.43 | Covalent Bonding | - | 32 |
| Comp.Ex.28 | No Covalent Bonding | - | <10 |
| Comp.Ex.29 | - | 1 | 20 |

(EXAMPLE 44)

The polystyrene-divinylbenzene copolymer induction enhancer-8 (product of Organo Corp., product number: AMBERLITE XAD-4) , measuring a bulk volume of 1 mL, and BCG (10 mg/mL) were intimately mixed in 1 mL physiological saline at 37 °C for 20 hours, so that BCG was physically adsorbed onto particle surfaces. After the intimate mixing, these particles were fully washed with physiological saline and again suspended in physiological saline. The thus-obtained induction enhancer, measuring a bulk volume of 100 μL, were packed in a sterilized tube.

BCG was not added in its individual form. The blood was added in the amount of 1.4 mL. Otherwise, the procedure of Example 1 was followed using blood from two healthy humans to determine the amount of IFN-γ induced. The results are shown in Table 15.

(COMPARATIVE EXAMPLE 30)

The procedure of Example 44 was followed, except that BCG was not added, to prepare particles. Using thus-obtained particles, the amount of IFN-γ induced was determined in the same manner as in Example 44. The results are shown in Table 15.

(COMPARATIVE EXAMPLE 31)

The induction enhancer was not added and the BCG-incorporated (1 mg/mL) blood was used in the amount of 1.5 mL. Otherwise, the procedure of Example 44 was followed to determine the amount of IFN-γ induced. The results are shown in Table 15.

Table 15

| | Induction Enhancer 8 Bulk Volume (100 μL) | Concentration of BCG (mg/mL) | Amount of IFN-γ Induced (pg/mL) | |
|---|---|---|---|---|
| | | | Blood | |
| | | | A | B |
| Ex.44 | Physical Adsorption | - | 396 | 109 |
| Comp.Ex.30 | No Physical Adsorption | - | <10 | <10 |
| Comp.Ex.31 | - | 1 | 109 | 29 |

(EXAMPLE 45)

The polystyrene-divinylbenzene copolymer induction enhancer-8, measuring a bulk volume of 1 mL, and BCG (10 mg/mL) were intimately mixed in 1 mL physiological saline containing 1 % formalin (neutral buffer formalin, product of Wako Pure Chemicals Industries, Ltd.) at 4 °C for 20 hours, so that BCG was physically adsorbed onto particle surfaces. Subsequent to the intimate mixing, these particles were fully washed with physiological saline. Thereafter, those particles, measuring a bulk volume of 100 μL, were packed in a sterilized tube (product of DIATRON Corp., for 1.5 mL use).
BCG was not added in its individual form. The blood was added in the amount of 1.4 mL. Otherwise, the procedure of Example 1 was followed to determine the amount of IFN-γ induced. The results are shown in Table 16.

(COMPARATIVE EXAMPLE 32)

The procedure of Example 45 was followed, except that BCG was not added, to determine the amount of IFN-γ induced. The results are shown in Table 16.

(COMPARATIVE EXAMPLE 33)

The induction enhancer-8 was excluded and the BCG-incorporated (1 mg/mL) blood was used in the amount of 1.5 mL. Otherwise, the procedure of Example 45 was followed to determine the amount of IFN-γ. The results are shown in Table 16.

Table 16

| | Induction Enhancer 8 Bulk Volume (100μL) | Concentration of BCG (mg/mL) | Amount of IFN-γ Induced (pg/mL) |
|---|---|---|---|
| Ex.45 | Physical Adsorption | - | 388 |
| Comp.Ex.32 | No Physical Adsorption | - | <10 |
| Comp.Ex.33 | - | 1 | 87 |

(EXAMPLE 46)

The induction enhancer-8 was treated in the same manner as in Example 45 so that BCG was physically adsorbed onto particle surfaces. The treated induction enhancer, measuring a bulk volume of 4 mL, was packed in a blood bag (product of Terumo Corp., separation bag for 200 mL use). Blood was collected from a healthy human to obtain venous blood containing 15 IU/mL of heparin. 50 mL of the venous blood was introduced into the blood bag. The blood bag contents were incubated while gently stirred at 37 °C for 24 hours. The amount of IFN-γ present in the blood was determined in the same manner as in Example 1. The results are shown in Table 17.

(COMPARATIVE EXAMPLE 34)

The induction enhancer-8 was used without the physical adsorption treatment'of BCG.' Otherwise, the procedure of Example 46 was followed. The results are shown in Table 17.

(COMPARATIVE EXAMPLE 35)

The induction enhancer-8 was excluded and the blood containing 1 mg/mL of BCG was used in the amount of 50 mL. Otherwise, the procedure of Example 46 was followed to determine the amount of IFN-γ. The results are shown in Table 17.

Table 17

| | Induction Enhancer 8 Bulk Volume (4 mL) | Concentration of BCG (mg/mL) | Amount of IFN-γ Induced (pg/mL) |
|---|---|---|---|
| Ex.46 | Physical Adsorption | - | 362 |
| Comp.Ex.34 | No Physical Adsorption | - | <10 |
| Comp.Ex.35 | - | 1 | 62 |

(EXAMPLE 47)

The procedure of Example 1 was followed, except that interleukin 2 and interleukin 12 present in the blood were quantitatively determined. The results are shown in Table 18.

(COMPARATIVE EXAMPLE 36)

The procedure of Example 47 was followed, except that BCG was not added. The results are shown in Table 18.

(COMPARATIVE EXAMPLE 37)

The induction enhancer-1 was not added and the BCG-incorporated blood was used in the amount of 1.5 mL. Otherwise, the procedure of Example 47 was followed. The results are shown in Table 18.

Table 18

| | Induction Enhancer 1 Bulk Volume (μL) | Concentration of BCG (mg/mL) | Amount of IL-2 Induced (pg/mL) | Amount of IL-12 Induced (pg/mL) |
|---|---|---|---|---|
| Ex.47 | 50 | 1 | 31 | 121 |
| Comp.Ex.36 | 50 | - | <6 | <8 |
| Comp.Ex.37 | - | 1 | 10 | 47 |

(EXAMPLE 48)

The procedure of Example 1 was followed, except that the BCG concentration was altered to 0.1 mg/mL and TGF-ß present in the blood was quantitatively determined. The results are shown in Table 19.

(COMPARATIVE EXAMPLE 38)

The induction enhancer-1 was not added and the BCG-incorporated blood plasma was used in the amount of 1.5 mL. Otherwise, the procedure of Example 48 was followed. The results are shown in Table 19.

Table 19

| | Induction Enhancer 1 Bulk Volume (μL) | Concentration of BCG (mg/mL) | Amount of TGF-β Induced (ng/mL) | | |
|---|---|---|---|---|---|
| | | | Blood | | |
| | | | A | B | C |
| Ex.48 | 50 | 0.1 | 80 | 101 | 63 |

Table 19 (continued)

| | Induction Enhancer 1 Bulk Volume (μL) | Concentration of BCG (mg/mL) | Amount of TGF-β Induced (ng/mL) | | |
|---|---|---|---|---|---|
| | | | Blood | | |
| | | | A | B | C |
| Comp.Ex.38 | - | 0.1 | 21 | 35 | 19 |

(EXAMPLE 49)

The procedure of Example 48 was followed, except that OK-432, in place of BCG, was used in the amount of 0.1 KE/mL. The results are shown in Table 20.

(COMPARATIVE EXAMPLE 39)

The induction enhancer-1 was not added and the OK-432 incorporated blood was used in the amount of 1.5 mL. Otherwise, the procedure of Example 49 was followed. The results are shown in Table 20.

Table 20

| | Induction Enhancer 1 Bulk Volume (μL) | Concentration of OK-432 (KE/mL) | Amount of TGF-β Induced (ng/mL) | | |
|---|---|---|---|---|---|
| | | | Blood | | |
| | | | A | B | C |
| Ex.49 | 50 | 0.1 | 71 | 111 | 71 |
| Comp.Ex.39 | - | 0.1 | 25 | 33 | 26 |

(EXAMPLE 50)

The procedure of Example 1 was followed, except that OK-432, in place of BCG, was used in the amount of 0.01 KE/mL and IL-10 was quantitatively determined. The results are shown in Table 21.

(COMPARATIVE EXAMPLE 40)

The induction enhancer-1 was not added and the OK-432 incorporated blood was used in the amount of 1.5 mL. Otherwise, the procedure of Example 50 was followed. The results are shown in Table 21.

Table 21

| | Induction Enhancer 1 Bulk Volume (μL) | Concentration of OK-432 (KE/mL) | Amount of IL-10 Induced (pg/mL) | | |
|---|---|---|---|---|---|
| | | | Blood | | |
| | | | A | B | C |
| Ex.50 | 50 | 0.01 | 560 | 285 | 421 |
| Comp.Ex.40 | - | 0.01 | 74 | 39 | 112 |

(EXAMPLES 51 AND 52)

The procedure of Example 1 was followed, except that BCG was added in the concentration of 0.1 mg/mL or 1 mg/mL. The results are shown in Table 22.

(COMPARATIVE EXAMPLE 41)

The procedure of Example 51 was followed, except that BCG was not added. The results are shown in Table 22.

(COMPARATIVE EXAMPLE 42)

The induction enhancer-1 was not added and the BCG-incorporated (0.1 mg/mL) blood was used in the amount of 1.5 mL. Otherwise, the procedure of Example 51 was followed. The results are shown in Table 22.

(COMPARATIVE EXAMPLE 43)

The induction enhancer-1 was not added and the BCG-incorporated (1 mg/mL) blood was used in the amount of 1.5 mL. Otherwise, the procedure of Example 52 was followed. The results are shown in Table 22.

Table 22

| | Induction Enhancer 1 Bulk Volume (μL) | Concentration of BCG (mg/mL) | Amount of IFN-γ Induced (pg/mL) | | |
|---|---|---|---|---|---|
| | | | Blood | | |
| | | | A | B | C |
| Ex.51 | 50 | 0.1 | 73 | 272 | 104 |
| Ex.52 | 50 | 1 | 376 | 350 | 401 |
| Comp.Ex.41 | 50 | - | <2 | 12 | 8 |
| Comp.Ex.42 | - | 0.1 | 9 | 93 | 36 |
| Comp.Ex.43 | - | 1 | 56 | 110 | 96 |

(EXAMPLES 53 AND 54)

The procedure of Example 1 was followed, except that OK-432, in place of BCG, was added in the concentration of 0.01 KE/mL or 0.1 KE/mL. The results are shown in Table 23.

(COMPARATIVE EXAMPLE 44)

The procedure of Example 53 was followed, except that OK-432 was not added. The results are shown in Table 23.

(COMPARATIVE EXAMPLE 45)

The induction enhancer-1 was not added and the OK-432 incorporated (0.01 KE/mL) blood was used in the amount of 1.5 mL. Otherwise, the procedure of Example 53 was followed. The results are shown in Table 23.

(COMPARATIVE EXAMPLE 46)

The induction enhancer-1 was not added and the OK-432 incorporated (0.1 KE/mL) blood was used in the amount of 1.5 mL. Otherwise, the procedure of Example 54 was followed. The results are shown in Table 23.

Table 23

| | Induction Enhancer 1 Bulk Volume (μL) | Concentration of OK-432 (KE/mL) | Amount of IFN-γ Induced (pg/mL) | | |
|---|---|---|---|---|---|
| | | | Blood | | |
| | | | A | B | C |
| Ex.53 | 50 | 0.01 | 79 | 355 | 128 |
| Ex.54 | 50 | 0.1 | 267 | 466 | 432 |
| Comp.Ex.44 | 50 | - | <2 | 12 | 8 |

Table 23   (continued)

| | Induction Enhancer 1 Bulk Volume (μL) | Concentration of OK-432 (KE/mL) | Amount of IFN-γ Induced (pg/mL) | | |
|---|---|---|---|---|---|
| | | | Blood | | |
| | | | A | B | C |
| Comp.Ex.45 | - | 0.01 | 8 | 94 | 59 |
| Comp.Ex.46 | - | 0.1 | 84 | 176 | 89 |

(EXAMPLE 55)

0.04 g (about 300 μL in terms of a bulk volume when packed in a 1.5 mL tube) of nylon wool (product of Wako Pure Chemicals Industries, Ltd.) was used as an induction enhancer-10. The BCG-incorporated blood was added in the amount of 1.2 mL. Otherwise, the procedure of Example 1 was followed. The results are shown in Table 24.

(EXAMPLE 56)

0.04 g (1 cm x 3 cm, about 300 μL in terms of a bulk volume) of a polyester nonwoven fabric (product of Japan Vilene Co., Ltd., product designation: EL-5600) was used as an induction enhancer-11. The BCG-incorporated blood was added in the amount of 1.2 mL. Otherwise, the procedure of Example 1 was followed. The results are shown in Table 24.

(EXAMPLE 57)

0.04 g (1 cmx3 cm, about 220 μL in terms of a bulk volume) of a polyester nonwoven fabric (product of Japan Vilene Co., Ltd., product designation: EW-7180) was used as an induction enhancer-12. TheBCG-incorporated blood wasaddedin the amount of 1.28 mL. Otherwise, the procedure of Example 1 was followed. The results are shown in Table 24.

(COMPARATIVE EXAMPLE 47)

The procedure of Example 55 was followed, except that BCG was excluded. The results are shown in Table 24.

(COMPARATIVE EXAMPLE 48)

The procedure of Example 56 was followed, except that BCG was excluded. The results are shown in Table 24.

(COMPARATIVE EXAMPLE 49)

The procedure of Example 57 was followed, except that BCG was excluded. The results are shown in Table 24.

(COMPARATIVE EXAMPLE 50)

The induction enhancer was excluded and the BCG-incorporated blood was used in the amount of 1.5 mL. Otherwise, the procedure of Example 55 was followed. The results are shown in Table 24.

Table 24

| | Induction Enhancer | Concentration of BCG (mg/mL) | Amount of IFN-γ Induced (pg/mL) |
|---|---|---|---|
| Ex.55 | Induction Enhancer 10 | 1 | 117 |
| Ex.56 | Induction Enhancer 11 | 1 | 381 |
| Ex.57 | Induction Enhancer 12 | 1 | 338 |
| Comp.Ex.47 | Induction Enhancer 10 | - | <10 |

Table 24 (continued)

| | Induction Enhancer | Concentration of BCG (mg/mL) | Amount of IFN-γ Induced (pg/mL) |
|---|---|---|---|
| Comp.Ex.48 | Induction Enhancer 11 | - | <10 |
| Comp.Ex.49 | Induction Enhancer 12 | - | <10 |
| Comp.Ex.50 | - | 1 | 37 |

(EXAMPLE 58)

Actinomyces S. nobilis (JCM 4274) obtained from Institute of Physical and Chemical Research was shake cultured at 30 °C for 40 hours in 100 mL of a starch-ammonium medium containing 0.2% yeast extract to obtain bacteria. The procedure of Example 1 was followed, except that such bacteria, in place of BCG, were added to blood in the dry weight of 1 mg/mL, to determine the amount of IFN-γ. The results are shown in Table 25.

(COMPARATIVE EXAMPLE 51)

The procedure of Example 58 was followed, except that the bacteria of actinomyces were excluded. The results are shown in Table 25.

(COMPARATIVE EXAMPLE 52)

The induction enhancer-1 was excluded and the actinomyces incorporated blood was used in the amount of 1.5 mL. Otherwise, the procedure of Example 58 was followed. The results are shown in Table 25.

Table 25

| | Induction Enhancer 8 Bulk Volume (μL) | Concentration of Bacteria (mg/mL) | Amount of IFN-γ Induced (pg/mL) |
|---|---|---|---|
| Ex. 58 | 50 | 1 | 161 |
| Comp.Ex.51 | 50 | - | <10 |
| Comp.Ex.52 | - | 1 | 32 |

(EXAMPLE 59)

As an induction enhancer-13 (gel particles of polyvinyl alcohol, particle diameter of about 1.1 mm), gel particles of polyvinyl alcohol-Fe (III) complex were prepared in accordance with "Complex gels of PVA and PAA" by Hiroshi Yokoi (Polymer Processing, Vol.40, No.11, 1991). The induction enhancer-13 was suspended in physiological saline. Subsequently, gel particles of polyvinyl alcohol-Fe (III) complex, measuringabulk volume of 300 μL, were packed in a sterilized tube for 1.5 mL use. The procedure of Example 1 was then followed, except that the blood was added in the amount of 1.2 mL. The results are shown in Table 26.

(COMPARATIVE EXAMPLE 53)

The procedure of Example 58 was followed, except that BCG was not added. The results are shown in Table 26.

(COMPARATIVE EXAMPLE 54)

The induction enhancer-13 was excluded and the BCG-incorporated blood was added in the amount of 1.5 mL. Otherwise, the procedure of Example 58 was followed. The results are shown in Table 26.

Table 26

| | Induction Enhancer 13 Bulk Volume (µL) | Concentration of BCG (mg/mL) | Amount of IFN-γ Induced (pg/mL) |
|---|---|---|---|
| Ex.59 | 300 | 1 | 129 |
| Comp.Ex.53 | 300 | - | <10 |
| Comp.Ex.54 | - | 1 | 42 |

## UTILITY IN INDUSTRY

Cytokines can be induced more effectively by the use of the cytokine-inducing material in accordance with the present invention than by conventional cytokine-inducing agents. Because the cytokine-inducing material of the present invention, when allowed to contact with blood or a blood component, can induce cytokines effectively, the cytokine-inducing material and cytokine-inducing instrument in accordance with the present invention can be suitably used to treat various diseases for which induction of cytokines is therapeutically effective.

## Claims

1.  A cytokine-inducing material characterized as containing a cytokine-inducing agent and a water-insoluble induction enhancer.

2.  The cytokine-inducing material as recited in claim 1, **characterized in that** said cytokine-inducing agent is fixed to said induction enhancer.

3.  The cytokine-inducing material as recited in claim 2, **characterized in that** said cytokine-inducing agent is fixed to said induction enhancer by physical adsorption.

4.  The cytokine-inducing material as recited in any one of claims 1 - 3, **characterized in that** said induction enhancer comprises a polymeric material.

5.  The cytokine-inducing material as recited in any one of claims 1 - 4, **characterized in that** said induction enhancer has a surface with a centerline average roughness of 0.2 - 10 µm.

6.  The cytokine-inducing material as recited in any one of claims 1 - 5, **characterized in that** said induction enhancer comprises at least one polymericmaterial selected from the group consisting of polystyrenes, acryl esters, polyesters, nylons, celluloses and polyvinyl alcohols.

7.  The cytokine-inducing material as recited in any one of claims 1 - 6, **characterized in that** said cytokine-inducing agent comprises bacteria or a substance derived from the bacteria.

8.  The cytokine-inducing material as recited in claim 7, **characterized in that** said cytokine-inducing agent comprises a acid-fast bacterium or a substance derived from the acid-fast bacterium.

9.  The cytokine-inducing material as recited in claim 7, **characterized in that** said cytokine-inducing agent comprises hemolytic streptococcal or a substance derived from the hemolytic streptococcal.

10. The cytokine-inducing material as recited in claim 7, **characterized in that** said cytokine-inducing agent comprises actinomycetes or a substance derived from the actinomycetes.

11. The cytokine-inducing material as recited in any one of claims 1 - 10, **characterized in that** said cytokine comprises at least one cytokine selected from the group consisting of interferon-γ, interleukin-2, interleukin-10, interleukin-12, tumor necrosis factor-α and transforming growth factor-β.

12. A cytokine-inducing instrument characterized as having a container and the cytokine-inducing material recited in any one of claims 1-11 and accommodated in the container.

13. The cytokine-inducing instrument as recited in claim 12, **characterized in that** said cytokine-inducing material accommodated in the container is allowed to contact with blood or a blood component.

FIG. 1

STANDARD LENGTH L

FIG. 2

STANDARD LENGTH L

FIG. 3

**EP 1 449 540 A1**

## INTERNATIONAL SEARCH REPORT

International application No.

PCT/JP02/11335

A. CLASSIFICATION OF SUBJECT MATTER
Int.Cl$^7$ A61K45/00, A61K47/48, A61K47/32, A61K47/34, A61K47/38, A61K35/74, A61P43/00

According to International Patent Classification (IPC) or to both national classification and IPC

B. FIELDS SEARCHED

Minimum documentation searched (classification system followed by classification symbols)
Int.Cl$^7$ A61K45/00, A61K47/00, A61K35/74, A61P43/00

Documentation searched other than minimum documentation to the extent that such documents are included in the fields searched

Electronic data base consulted during the international search (name of data base and, where practicable, search terms used)
JOIS(STN), CAPLUS(STN), BIOSIS(STN), MEDLINE(STN), EMBASE(STN), WPI

C. DOCUMENTS CONSIDERED TO BE RELEVANT

| Category* | Citation of document, with indication, where appropriate, of the relevant passages | Relevant to claim No. |
|---|---|---|
| X<br>Y | JP 61-277628 A (ASAHI KASEI KOGYO KABUSHIKI KAISHA),<br>08 December, 1986 (08.12.86),<br>(Family: none) | 1-4,6-13<br>1-13 |
| X<br>Y | EP 147689 A2 (ASAHI KASEI KOGYO KABUSHIKI KAISHA),<br>10 July, 1985 (10.07.85),<br>& JP 60-120821 A          & JP 60-252423 A<br>& JP 61-85317 A          & JP 61-87671 A<br>& JP 61-93121 A          & JP 61-93122 A<br>& US 4839290 A | 1-4,6-13<br>1-13 |
| X<br>Y | Moto ABE, "LPS Koteika Beads ni yoru Koshuyo Koka",<br>Nichigai Kaishi, 1991, Vol.92, No.6, pages 627 to<br>635, full text; particularly, the abstract, page<br>628, left column, line 8 to right column, line 1;<br>page 632, left column, lines 17 to 38; page 633,<br>left column, lines 13 to 34 | 1-4,6-13<br>1-13 |

☒ Further documents are listed in the continuation of Box C.   ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>09 January, 2003 (09.01.03) | Date of mailing of the international search report<br>12 February, 2003 (12.02.03) |
|---|---|
| Name and mailing address of the ISA/<br>Japanese Patent Office | Authorized officer |
| Facsimile No. | Telephone No. |

Form PCT/ISA/210 (second sheet) (July 1998)

26

**EP 1 449 540 A1**

**INTERNATIONAL SEARCH REPORT**

| | International application No. |
|---|---|
| | PCT/JP02/11335 |

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | BONFIELD, T.L. et al., "Cytokine and growth factor production by monocytes/macrophages on protein preadsorbed polymers.", J.Biomed.Mater.Res., 1992, Vol.26, No.7, pages 837 to 850<br>Full text; particularly, abstract; page 838, lines 4 to 11; tables II to IV; Figs. 1 to 2 | 1-4,6,11-13<br>1-13 |
| X<br>Y | BONFIELD, T.L. et al., "Plasma protein adsorbed biomedical polymers: activation of human monocytes and induction of interleukin 1.", J.Biomed.Mater. Res., 1989, Vol.23, No.6, pages 535 to 548<br>Full text; particularly, tables I to II | 1-4,6,11-13<br>1-13 |
| X<br>Y | ANDERSON, J.M. et al., "Protein adsorption and cellular adhesion and activation on biomedical polymers.", Int.J.Artif.Organs., 1990, Vol.13, No.6, pages 375 to 382 | 1-4,6,11-13<br>1-13 |
| X<br>Y | MILLER, K.M. et al., "Human monocyte/macrophage activation and interleukin 1 generation by biomedical polymers.", J.Biomed.Mater.Res., 1988, Vol.22, No.8, pages 713 to 731<br>Full text; particularly, Figs. 1 to 6; tables I to V | 1-4,6,11-13<br>1-13 |
| X<br>Y | JP 61-113465 A (Biomedical Kabushiki Kaisha), 31 May, 1986 (31.05.86),<br>Full text; particularly, example 1<br>(Family: none) | 1-4,6,11-13<br>1-13 |
| Y | Makoto YAMADA et al., "Cytokine Sanseino kara mita BRM no Sayo Kojo to Tekiosho-rei Sentaku Kijun ni Kansuru Kenkyu (II) Masshoketsu Zenketsu o Mochiita BRM Shigeki niyoru Sytokine Sanseiryo no Kento", Gifudai Iki, 1995, Vol.43, No.1, pages 166 to 177 | 1-13 |
| Y | Yasuhito, A. et al., "The endogenous induction of tumor necros is factor serum (TNS) for adjuvant postoperative immunotherapy of cancer. -changes in immunological markers of the blood-", Japanese Journal of Surgery, 1990, Vol.20, No.1, pages 19 to 26 | 1-13 |
| Y | Akira HAYASHI, "Gan Men'eki Ryoho no Atarashii Tenkai - Shoki Men'eki to Kakutoku Men'eki no Kakehashi to shite no BCG-CWS", Molecular Medicine, 1999, Vol.36, special issue, pages 220 to 229<br>Full text; particularly, page 223, right column, line 5 to page 224, left column, line 1 | 1-13 |
| Y | Yoshiki RYOMA, "Hi Tokuiteki Ko Akusei Shuyozai Sonogo no Tenkai OK-432 (Picibanil), Sonogo no Tenkai", Biotherapy, 2000, Vol.14, No.9, pages 877 to 885 | 1-13 |

Form PCT/ISA/210 (continuation of second sheet) (July 1998)

27

**INTERNATIONAL SEARCH REPORT**

International application No.

PCT/JP02/11335

C (Continuation).  DOCUMENTS CONSIDERED TO BE RELEVANT

| Category* | Citation of document, with indication, where appropriate, of the relevant passages | Relevant to claim No. |
|---|---|---|
| Y | FUJIMOTO, T. et al., "Streptococcal preparation OK-432 is a potent inducer of IL-12 and a T helper cell 1 dominant state.", J.Immunol., 1997, Vol.158, No.12, pages 5619 to 5626 | 1-13 |
| Y | ABE, Y. et al., "Wound healing acceleration of a novel transforming growth factor-β inducer, SEK-1005.", Eur.J.Pharmacol., 2000, Vol.408, No.2, pages 213 to 218 | 1-13 |
| Y | JP 6-209992 A (Sekisui Chemical Co., Ltd.), 02 August, 1994 (02.08.94), Full text; particularly, Claim 1; page 3, column 4, Par. No. [0016] to page 4, column 5, Par. No. [0027] (Family: none) | 1-13 |
| Y | Kazuo NIIMURA et al., "Somen Sakusan Cellulose Beads no Shuyo Kaishi Inshi Yuki Sayo", The Japanese Journal of Artificial Organs, 1993, Vol.22, No.5, pages 1233 to 1237, full text; page 1234, left column, line III.1., page 1235, left column, line 2 to page 1236, last part | 1-13 |
| Y | Ayumi MITO et al., "Gosei Kobunshi Zairyo no IL2 Kassei oyobi IL2 Receptor Hatsugen ni oyobosu Eikyo", The Japanese Journal of Artificial Organs, 1991, Vol.20, No.1, pages 235 to 240 | 1-13 |
| Y | Hiroshi MIYAZAKI et al, "Men'eki Tanto Saibo Kino Seigyo ni oyobosu Kobunshi Zairyo no Macro Kozo Koka -IL-2, IL-1 Kassei no Sokutei-", The Japanese Journal of Artificial Organs, 1991, Vol.20, No.1, pages 241 to 246 | 1-13 |
| Y | MILLER, K.M. et al., "Characterization of biomedical polymer-adherent macrophages: interleukin 1 generation and scanning electron microscopy studies.", Biomaterials., 1989, Vol.10, No.3, pages 187 to 196 | 1-13 |
| Y | LUGER, A. et al., "Blood-membrane interaction in hemodialysis leads to increased cytokine production", Kidney Int., 1987, Vol.32, No.1, pages 84 to 88 | 1-13 |
| Y | JP 7-67955 A (Sekisui Chemical Co., Ltd.), 14 March, 1995 (14.03.95), (Family: none) | 1-13 |
| Y | JP 9-87181 A (Sekisui Chemical Co., Ltd.), 14 March, 1997 (14.03.97), (Family: none) | 1-13 |

Form PCT/ISA/210 (continuation of second sheet) (July 1998)

**EP 1 449 540 A1**

**INTERNATIONAL SEARCH REPORT**

International application No.

PCT/JP02/11335

C (Continuation). DOCUMENTS CONSIDERED TO BE RELEVANT

| Category* | Citation of document, with indication, where appropriate, of the relevant passages | Relevant to claim No. |
|---|---|---|
| Y | JP 7-96031 A (Sekisui Chemical Co., Ltd.), 11 April, 1995 (11.04.95), (Family: none) | 1-13 |

Form PCT/ISA/210 (continuation of second sheet) (July 1998)

29